(19) Europäisches Patentamt
European Patent Office
Office européen des brevets

(11) **EP 3 005 942 B1**

(12) **EUROPEAN PATENT SPECIFICATION**

(45) Date of publication and mention
of the grant of the patent:
**03.12.2025 Bulletin 2025/49**

(21) Application number: **14188200.1**

(22) Date of filing: **08.10.2014**

(51) International Patent Classification (IPC):
*A61B 5/0537* (2021.01)        *A61B 8/08* (2006.01)
*A61B 5/145* (2006.01)        *A61B 5/1477* (2006.01)

(52) Cooperative Patent Classification (CPC):
**A61B 5/0537; A61B 5/14542; A61B 5/1477;**
A61B 5/25; A61B 5/291

(54) **Method for the detection and characterisation of bubbles in liquids and device therefor, resp.system**

Verfahren zur Detektion und Charakterisierung von Blasen in Flüssigkeiten und Vorrichtung dafür, zugehöriges System

Procédé pour la détection et la caractérisation de bulles dans des liquides et dispositif à cet effet, système resp.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.04.2016 Bulletin 2016/15**

(60) Divisional application:
**21207579.0 / 4 018 924**

(73) Proprietor: **ARISTOTLE UNIVERSITY OF THESSALONIKI-**
**Research Committee**
**54636 Thessaloniki (GR)**

(72) Inventors:
- **Karapantsios, Theodoros**
  **GR-57013**
  **Oreokastro, Thessaloniki (GR)**
- **Evgenidis, Sotiris**
  **57010 Chortiatis Thessalonikis (GR)**
- **Zacharias, Konstantinos**
  **GR 55337 Triandria, Thessaloniki (GR)**
- **Mesimeris, Theodoros**
  **54622 Thessaloniki (GR)**

(74) Representative: **Petsis, Christos**
**4-6, Kyparissias**
**542 49 Thessaloniki (GR)**

(56) References cited:
**WO-A1-99/65390**

- **MOHAN M ET AL: "Investigation of a Bubble Detector based on Active Electrolocation of Weakly Electric Fish", JOURNAL OF PHYSICS: CONFERENCE SERIES, INSTITUTE OF PHYSICS PUBLISHING, BRISTOL, GB, vol. 434, no. 1, 18 April 2013 (2013-04-18), pages 12088, XP020240827, ISSN: 1742-6596, DOI: 10.1088/ 1742-6596/434/1/012088**
- **NEBUYA S ET AL: "Detection of emboli in vessels using electrical impedance measurements-phantom and electrodes; Emboli detection by electrical impedance", PHYSIOLOGICAL MEASUREMENT, INSTITUTE OF PHYSICS PUBLISHING, BRISTOL, GB, vol. 26, no. 2, 1 April 2005 (2005-04-01), pages S111 - S118, XP020092153, ISSN: 0967-3334, DOI: 10.1088/0967-3334/26/2/011**
- **NEBUYA S ET AL: "Estimation of the size of air emboli detectable by electrical impedance measurement", MEDICAL & BIOLOGICAL ENGINEERING & COMPUTING, SPRINGER, BERLIN, DE, vol. 42, no. 1, 1 January 2004 (2004-01-01), pages 142 - 144, XP019834637, ISSN: 1741-0444**
- **YUNUS F R M ET AL: "Simulation study of electrode size in air-bubble detection for dual-mode integrated electrical resistance and ultrasonic transmission tomography", POWDER TECHNOLOGY, vol. 256, 1 April 2014 (2014-04-01), pages 224 - 232, XP028830451, ISSN: 0032-5910, DOI: 10.1016/ J.POWTEC.2014.02.001**

## Description

FIELD OF THE INVENTION

The invention relates to a method for the detection and characterization of bubbles in liquids and device for carrying out said method.

BACKGROUND OF THE INVENTION

The presence of bubbles in liquids is encountered in a variety of industrial processes covering chemical and petroleum processing, oil and gas extraction and transportation, and nuclear power generation, up to the human's blood circulation during Decompression Sickness notably in astronauts, scuba divers and metro workers, which may cause undesired effects. A reliable, non-invasive bubbles detection and characterization is thus of particular importance both for the control and improvement of industrial processes, and also for the protection of human health.

Existing non-invasive techniques for the detection and characterization of bubbles in liquids can be divided into three main categories: optical, acoustic and electrical.

PRIOR ART

The optical measuring techniques are widely used to measure the bubble size, including photographic techniques and techniques based on scattering and diffraction of light. The main disadvantages of optical techniques are two: their use is limited to transparent liquids and measurement of low volumetric gas fractions. In addition, the imaging techniques including X-Ray, Gamma Ray, MRI, yet provide excellent spatial resolution but the presence of intense radiation and magnetic fields, the use of cryogenic liquids, such as liquid helium and liquid nitrogen, and slow time response make them impractical in industrial applications.

As regards the acoustic techniques, they are divided into passive and active. Passive techniques do not have wide application because it is very difficult to extract information from the acoustic wave generated from gas phase flow. In that sense, the pressure measurement techniques are considered passive as well. The LG01 by SENSIRION, US-6,813,944 B2 is a non-invasive passive pressure measurement sensor, not a complete integrated system. However, it is employed in limited industrial applications due to a low flow rate measurement range. The detection range is limited to high volumetric gas fraction changes only, while the threshold of detection is unspecified. Also, said LG01 system has the drawback not to provide any information regarding the bubble size. Active acoustic techniques, on the other hand, are based on measuring the propagation speed and attenuation of acoustic waves in liquids. The presence of bubbles affects both the speed and attenuation, and this effect is more pronounced with the increase of volumetric gas fraction. Their main drawback is the lack of reliable measurements for volumetric gas fraction values higher than 0,1%, resulting in a very high attenuation and making active acoustic techniques impractical for industrial applications. Even when reliable measurements are conducted, the lack of rigorous mathematical modelling as well as numerical solving limitations prohibit the interpretation of acoustic signals in terms of bubble size detection and characterization. Besides, the necessary hardware is quite complex for accurate measurements.

Referring to US 7,661,293 & 7,661,294, the AD-101 by Cosense Inc. is an active acoustic measurement sensor, but not a complete integrated system however, based on transmitting and receiving ultrasonic waves. It is applied to detect bubbles non-invasively, but its sensitivity is unspecified. Moreover, it does not provide information regarding the bubble size and it cannot be employed for pipe diameters larger than 25 mm. Also, the continuous wave (CW) or pulsed wave (PW) medical ultrasound systems as well as Doppler techniques are sorted in active acoustic techniques category. However, these ultrasound systems provide mainly qualitative information, while quantitative estimation of bubble size is problematic because of several technical drawbacks, which is thus unsatisfactory.

The said third category is directed to the electrical techniques, which are based on measuring the impedance, the capacitance or equivalently the electric permittivity, or the conductance - or inversely the resistance- of the liquid. Referring to WO 99/65390, is a non-invasive diagnostic system for the monitoring of physiological parameters, based on measuring and processing the bio-impedance. As regards bubbles detection, said system claims the indication of appearance of gas bubbles in blood during decompression, but does not provide any estimation of their size or corresponding gas volumetric fraction. The signal processing for bubbles indication includes the frequency components from about 0.01 Hz to about 0.4Hz. This frequency range is attributed to breathing and the spectral content is very unlikely to be affected by the presence of bubbles. As far as is known to the inventors knowledge of the present invention, the presence of bubbles in blood contain primarily frequency components higher than 0.4Hz, located both in time and frequency, that require advanced signal processing techniques, e.g. continuous and discrete wavelets transform, spectrogram analysis, etc. Consequently, said system has the drawback to include only the Fast Fourier Transform which is insensitive in detecting impedance wave undulations located in time and does not support any additional signal processing methods. As regards the conventional electrical techniques, when applied in industrial applications, provide reliable results for high values of volumetric gas fraction, i.e. above 10%, which again does not provide a comprehensive technique leaving the problem unsolved for lower fractions.

## AIM OF THE INVENTION

The present invention aims at remedying the lacks set out above by providing a solution to the problem including for both the former higher fractions and the latter lower fractions tending to a complete integrated system.

## SUMMARY OF THE INVENTION

For that purpose, there is provided according to the invention an electrical impedance spectroscopy method for non-invasive bubbles detection as well as estimation of their size and corresponding gas volumetric fraction in the human body of astronauts, scuba divers, metro workers and patients treated in decompression chambers, comprising the steps of :

    a) attaching at least two non-invasive sensors, particularly electrodes to a human body, notably including Electro-CardioGraphy (ECG) or ElectroEncephaloGraphy (EEG) pads;
    b) acquiring electrical impedance measurements by means of a data acquisition card of high sampling rate and resolution;
    c) real time monitoring by means of a PC, storing and digital processing of acquired signals.

Thanks to the invention, there is thus provided an innovative, highly sensitive and accurate, non-invasive electrical impedance spectroscopy technique.

Particularly, the method proposed according to the invention allows detecting accurately for the first time even the presence of a few bubbles corresponding to extremely low volumetric gas fraction values, up to less than 0,1%. Also, the high measurement sensitivity of 0,001% in conjunction with the appropriate signal analysis provides valuable qualitative and quantitative information on bubble size characteristics. Furthermore, the use of advanced signal processing *techniques* allows for correlating bubble size with the spectral content of acquired signals.

According to a particular embodiment of the invention, said method comprises the step of a non-intrusive bubbles detection in blood flow generated by extracorporeal circulation equipment utilized during heart surgery, by means of at least two flush-mounted ring electrodes that are provided for conducting electrical measurements.

According to a preferred embodiment of the invention, said method comprises the steps of a non-intrusive bubbles detection, bubble size and gas volumetric fraction estimation as well as flow regime transition identification in gas-liquid two-phase flows found in several industrial applications, by means of a set of pipes that is incorporated for fluids' transport and wherein the presence of gas phase is of great significance, such as in the food industry, petroleum lines, product line of polymers, comparing to the previous ones, wherein at least two flush-mounted ring, plate or any other type of electrodes depending on the pipe cross-section circular, rectangular, etc. are incorporated for conducting electrical measurements.

According to a further embodiment of the invention, said method comprises the steps of non-intrusive solid particles detection, particles size and solid concentration estimation in solid-liquid two-phase flows found in several industrial applications, such as in food industry.

According to a still further embodiment of the invention, said method comprises the step of non-intrusive estimation of the concentration of each liquid phase in a flow consisting of two immiscible liquids.

According to a yet further embodiment of the invention, said method is for the study of emulsion stability and foam drainage that interests food industry and cosmetics.

According to another embodiment of the invention, said method consists of a crack or/and air pocket detection as well as relevant humidity estimation in construction materials, wherein two or more plate electrodes are placed on the surface of the material under test for conducting electrical measurements.

According to yet another embodiment of the invention, said method consists of an accurate, multi-frequency measurement of liquids' electrical conductivity. In this case, two plate electrodes are immersed in the test liquid and placed opposite each other for conducting electrical measurements.

This invention also relates to a novel electrical impedance spectroscopy device for carrying out said method that is based on measuring non-invasively with exceptional accuracy and sensitivity the electrical impedance variation of two-phase mixtures, wherein liquid is a conductive phase, and gas a non-conductive phase. For this, preliminary frequency scanning identifies the proper frequency value of the applied AC excitation signal in terms of measurement sensitivity and accuracy. Additionally, innovative signal acquisition, as hardware, and digital signal processing, as software methods are applied to improve measurement sensitivity several orders of magnitude compared with conventional electrical techniques. Said device according to the invention is remarkable in that detection means are provided therein for a non-invasive bubbles detection as well as estimation means for the estimation of their size and corresponding gas volumetric fraction, notably in the human body of astronauts, scuba divers, metro workers and patients treated in decompression chambers. Said device comprises :

- at least two non-invasive sensors, particularly electrodes for being attached to a human body, such as Electro-CardioGraphy (ECG) or Electro-EncephaloGraphy (EEG) pads;
- a data acquisition card of high sampling rate and resolution for acquiring electrical impedance measurements;
- a PC for real time monitoring, storing and digital processing of acquired signals.

According to a particular embodiment of said device according to the invention, at least two flush-mounted ring electrodes are provided for conducting electrical measurements for non-intrusive bubbles detection in blood flow generated by extracorporeal circulation equipment utilized during heart surgery.

According to a more particular embodiment of the device according to the invention, at least two flush-mounted ring, plate or any other type of electrodes depending on the pipe cross-section circular, rectangular, are provided for conducting electrical measurements for non-intrusive bubbles detection, bubble size and gas volumetric fraction estimation as well as flow regime transition identification in gas-liquid two-phase flows found in several industrial applications. In addition, a set of pipes is provided therein for fluids' transport and wherein the presence of gas phase is of great significance, notably in food industry, petroleum lines, product line of polymers, comparing to the previous claims. Therein at least two flush-mounted rings, plate or any other type of electrodes depending on the pipe cross-section circular, rectangular, etc. are provided for conducting electrical measurements.

According to an even more particular embodiment of the device according to the invention, it includes means for non-intrusive solid particles detection, particles size and solid concentration estimation in solid-liquid two-phase flows found in several industrial applications, e.g. food industry.

According to a yet more particular embodiment of the device according to the invention, it includes means for a non-intrusive estimation of the concentration of each liquid phase in a flow consisting of two immiscible liquids.

According to a still more particular embodiment of the device according to the invention, it includes means for the study of emulsion stability and foam drainage that interests food industry and cosmetics.

According to a embodiment of the device according to the invention, it includes means for crack or/and air pocket detection as well as relevant humidity estimation in construction materials, wherein two or more plate electrodes are placed on the surface of the material under test for conducting electrical measurements.

According to a particularly remarkable embodiment of the device according to the invention, it includes means for accurate, multi-frequency measurement of liquids' electrical conductivity. In this case, two plate electrodes are immersed in the test liquid and placed opposite each other for conducting electrical measurements.

Further features and properties of the device and the method according to the invention are defined in the further appended subclaims.

Further details of the device and the anchoring method will emerge from the following detailed description of some embodiments of the invention, which are illustrated with the aid of the attached drawings.

BRIEF DESCRIPTION OF THE DRAWINGS

FIG. 1 presents the design logic of the electrical impedance technique applying the voltage source excitation mode for the detection and characterization of bubbles in liquids.

FIG. 2 shows the electrical equivalent of the impedance technique applying the voltage source excitation mode.

FIG. 3 presents the design logic of the electrical impedance technique applying the current source excitation mode for the detection and characterization of bubbles in liquids.

FIG. 4 shows the electrical equivalent of the impedance technique applying the current source excitation mode.

FIG. 5 demonstrates the circuit schematic of the novel Howland modified Voltage Controlled Current Source (VCCS), for AC voltage sources as an input.

FIG. 6 depicts a perspective cut-away, cross section view of the flush mounted ring electrodes to the inner walls of a cylindrical dielectric (non electrical conductive) pipe section.

DESCRIPTION

The proposed invention is categorized to the electrical techniques measuring the electrical impedance. The electrical impedance technique consists of the hardware and the corresponding program code as a software for digital processing of measurements. The electrical quantities that are measured and taken into account are the resistance -or inversely the conductance- and the reactance or both of them, i.e. impedance. It is based on measuring the variation of electrical impedance of the two-phase mixture, i.e. liquid being a conductive phase, resp. gas as a non-conductive phase, by applying a high frequency alternating electric current (AC) or voltage via properly designed electrodes.

Electrodes geometry varies with the application. When the technique is applied in-vitro for bubbles detection inside a pipe, then ring, plate or any other type of electrodes may be used depending on the pipe cross-section, e.g. circular, rectangular, etc.. In any case, the electrodes are flush mounted to the inner walls of the pipe in order to provide non-intrusive measurements avoiding any undesired flow disturbance. The proper selection of electrodes' dimensions and separation distance is a matter of concern and thus is taken seriously into account, as these two parameters affect strongly the measuring volume inside the pipe as well as the spatial resolution. As concerns in-vivo applications of the impedance technique, e.g. for bubbles detection inside the human blood flow when an incident of DCS takes place, standard ElectroCardioGraphy (ECG) or ElectroEncephaloGraphy (EEG) electrode pads are applied above the human skin providing non-invasive measurements.

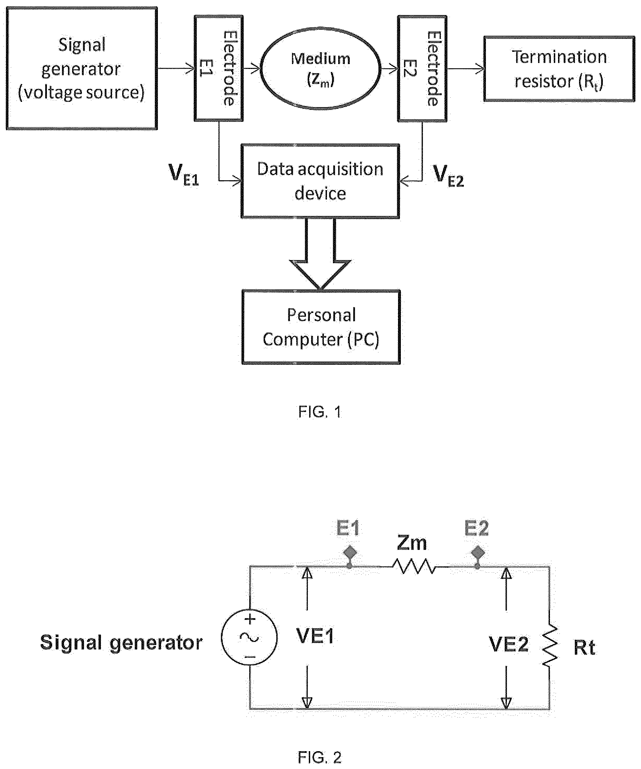

As mentioned above, there are two applicable excitation modes: voltage source, when the applied voltage is set at a defined value (voltage control), and current source, when the applied current is set at a defined value (current control). Fig. 1 demonstrates the design logic for measuring the electrical impedance of a gas/liquid mixture $Z_m$, or inversely the electrical admittance $Y_m$, applying the voltage source excitation mode. Moreover, Fig. 2 shows the electrical equivalent of the measurement system presented in Fig. 1. $V_{E1}$ is the input voltage that excites electrically the medium, connected to the input electrode E1 generating an input current passing through the medium. The hardware provides the optimum adjustment of AC voltage -or AC current when a current source is applied- in terms of amplitude and frequency employed to the respective electrodes which are placed either inside a pipe or above the human skin. Regardless the excitation mode -voltage or current- and application - in-vitro or in-vivo -, preliminary testing reveals the proper excitation frequency and amplitude that provide the highest measurement sensitivity and accuracy. Also, the hardware of the novel electrical impedance technique allows for employing different waveform types of excitation signal to the electrodes -continuous: sinusoidal, square, triangular, etc., or pulsed-, depending on the application requirements. The input current generated by the excitation voltage $V_{E1}$ creates a voltage drop due to the finite, but time variable, impedance $Z_m$ of the medium, and exits the section from the output electrode E2. The output electrode is connected to the one end of the termination resistance Rt, while the other end of the resistance is connected to signal generator (common voltage point). If needed, the common point could be grounded. The voltage across the termination resistance is the measured output voltage $V_{E2}$. $Z_m$ and $R_t$ constitute a voltage divider and the ratio $k$ of the voltages is:

$$k = \frac{V_{E2}}{V_{E1}} = \frac{R_t}{Z_m + R_t} \qquad (1)$$

Solving for $Z_m$, it is obtained:

$$Z_m = \left(k^{-1} - 1\right) R_t \qquad (2)$$

$Z_m$ is the equivalent impedance as measured by the two electrodes. Equation 1 can be written in the following complex exponential form:

$$\frac{R_t}{|Z_m| e^{j\varphi_m} + R_t} = |k| e^{j\varphi_k} \qquad (3)$$

where $|Z_m|$ and $\varphi_m$ represent the magnitude and the phase of $Z_m$ respectively and suchlike $|k|$ and $\varphi_k$ are the magnitude and the phase of $k$. It stands from the electric circuit's theory that any impedance can be resolved in Cartesian form into a real and an imaginary component $Z = R + jX$, i.e. resistance and reactance respectively. Therefore, any impedance can be mathematically represented by a complex number and the imaginary component is modelled depending on the sign by either an inductor or a capacitor, which are frequency selective components. Consequently, the voltage ratio $k$ will be a complex number and a function of frequency. In a conductive medium, the bubbles affect the resistance of the medium and any variation of the resistance, due to bubbles presence, is compared with the reactive component of the impedance. Therefore, the effect of bubbles on the magnitude of the impedance $Z_m$ is reduced and as a result the bubble detection sensitivity deteriorates. In order to obtain the maximum possible bubble detection sensitivity, the reactance (imaginary part) of $Z_m$ has to be ideally zero. In practice, if the reactance $X$ (imaginary component) is small compared to the resistance $R$ (real component), then the phase shift of the output relative to the input current can be neglected. This requirement is fulfilled when the reactance is less than 1/10 of the resistance:

$$\frac{X}{R} \leq \frac{1}{10} \qquad (4)$$

Therefore, based on Equation (4) the magnitude of the impedance is:

$$Z = \sqrt{R^2 + X^2} = \sqrt{R^2 + \frac{R^2}{100}} = R\sqrt{1.01} = 1.0049R \approx R \qquad (5)$$

From Equation (5) it results that if the requirement of Equation (4) is fulfilled, then the impedance is transformed to resistance and Equation 2 is reduced to:

$$R_m = \left(k^{-1} - 1\right)R_t \qquad (6)$$

Equivalently, the corresponding phase shift requirement can be written as:

$$\varphi \leq \tan^{-1}\left(\frac{X}{R}\right) = \tan^{-1}\left(\frac{1}{10}\right) \qquad (7)$$

$$-6° \leq \varphi \leq 6° \qquad (8)$$

It is underlined that the phase response analysis is independent of the excitation technique and the kind of source -voltage or current- as well. There is a possibility of some imaginary component existence, even in a purely resistive medium, due to stray capacitances of the connection wires or electrodes. In this case, considering that an air bubble is dielectric, the maximum bubble detection sensitivity is accomplished when there is no phase shift in the excitation current. Therefore, if needed, a frequency scanning can be conducted to identify the phase response of the liquid with respect to frequency. This is accomplished by applying digital signal analysis and the phase shift can be calculated for any discrete frequency, revealing the phase response of the liquid. This allows setting the excitation frequency of the signal generator in the frequency range that satisfies the phase shift requirement of Equation (8), therefore indicating the range of maximum detection sensitivity.

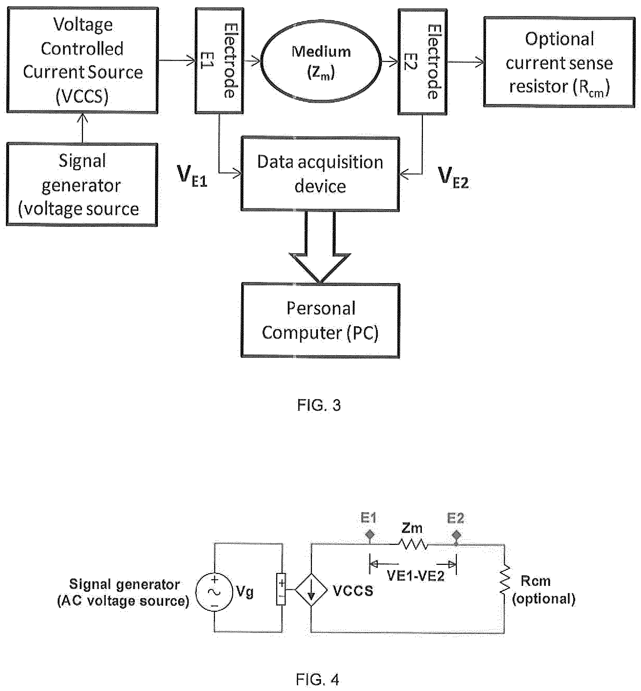

The current source excitation mode is accomplished by applying an alternating current Voltage Controlled Current Source (VCCS). The VCCS is located between the voltage source and the electrode E1, as shown in Fig. 3. Fig. 4 shows the electrical equivalent of the measurement set-up shown in Fig. 3. For the needs of the excitation, a proper AC current source (VCCS) of the desired frequency and amplitude is applied. $V_g$ is the output voltage of the signal generator, $V_m = V_{E1} - V_{E2}$ is the differential voltage measured across the electrodes E1 and E2 caused by the excitation current of the VCCS, $Z_m$ is the measured impedance between the two applied electrodes and $R_{cm}$ is an optional resistor for current sensing. The VCCS is a component that converts any type of voltage source into a current source. The current is controlled by the voltage applied at the input. This feature is the great advantage of VCCS compared with a current source generator. Instead of using a current generator where the available waveform type would only be sinusoidal, the VCCS can convert any arbitrary waveform voltage. For example, continuous waves such as sinusoidal, square, triangular, etc., or pulse waves of any voltage amplitude and frequency can be converted. Although the vast majority of applications for the detection and characterization of bubbles in liquids would require a sinusoidal waveform, any other waveform type can be employed and tested. Since the output of the VCCS is current and the input is voltage, therefore the transfer function, which is the output over the input, is in terms of $\Omega^{-1}$. These components are also called as transconductance devices and the most commonly used are the transconductance amplifiers (TA). The transconductance amplifier is like a typical voltage amplifier but the amplified output is a current. It is an amplifier because the magnitude of the transfer function is

$|H_{TA}| = \frac{I_o}{V_i} > 1$ , where $I_o$ is the output current and $V_i$ is the input voltage. For the application of the impedance technique in liquids, the excitation current does not have to be greater than a few mA. Also, it shall be taken into account that many biomedical devices operate with a stimulation current of 1mA RMS or less, e.g. 0,1 mA, as the present electrical impedance technique intends to be applied for bubbles detection in humans' bloodstream as well. As a result, the output of the voltage source would be in the order of a few mV, which is not practically possible. The practical implementation of the VCCS requires the current output to be in mA and the voltage input in V, therefore the magnitude of the transfer function of VCCS $|H_{VCCS}|$ would be far less than one :

$$| H_{VCCS} |= \frac{I_o}{V_i} = \frac{mA}{V} << 1 \qquad (9)$$

Hence, the VCCS is not a TA but a transconductance attenuator. After worldwide survey, there is no commercial off-the-shelf device that fulfils the requirements of VCCS as a transconductance attenuator, in terms of attenuation, frequency response, distortion, noise, etc. Therefore, a custom made VCCS circuit is designed in order to fulfil the requirements for the detection and characterization of bubbles in liquids. A practical value of the $|H_{VCCS}|$ or gain would be:

$$| H_{VCCS} |= \frac{1mA}{1V} = 0.001 \qquad (10)$$

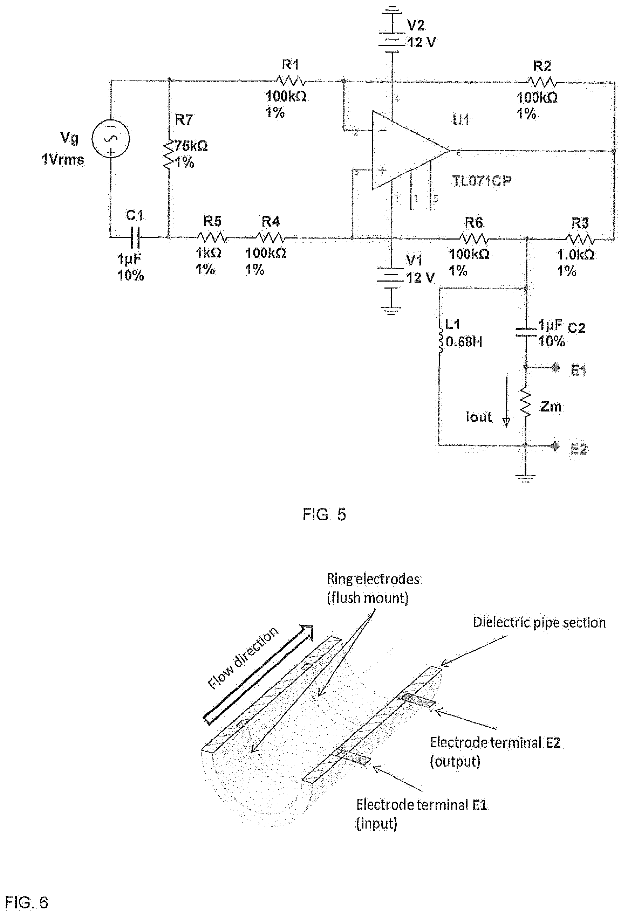

For example, 1V of the voltage source is transformed (converted) into 1mA current excitation passing through the electrode E1. In the case that it is considered necessary, the VCCS gain could be adjusted (increased or decreased) at any desired value. It is known from electrical circuits' theory that the current source has very high internal impedance (theoretically infinite) while the voltage source has low internal impedance (theoretically zero). Therefore the positive and the negative inputs of an operational amplifier can be used to make a current source. The VCCS is essentially a high impedance current source, and many alternative designs of current sources and VCCS exist for testing other devices and materials, or driving sensors. Most of the designs are for current sources controlled by a DC voltage source. But for the demanding application of the detection and characterization of bubbles in liquids, both in-vitro and in-vivo, where the practical problems involved are not so simple or/and obvious, a versatile and reliable alternating current source (AC current) is needed, with high output impedance and wide frequency range. For these tasks, a novel, modified and improved alternating current VCCS, based on Howland Current circuit is designed, as shown in Fig. 5. The gain is set by $R_3$ and ratio of $R_1/R_2$ (which is typically 1/1). In the new improved design, the ratio of $(R_5+R_4)/(R_6+R_3)$ should be matched with $R_1/R_2$ for proper biasing of the operational amplifier (Op-amp), so:

$$\frac{R_5 + R_4}{R_6 + R_3} = \frac{R_1}{R_2} \qquad (11)$$

The gain then is:

$$| H_{VCCS} |= \frac{I_{out}}{V_g} = \frac{1}{R_3} \qquad (12)$$

Consequently, low values for $R_3$ can be used and have all the other resistors high in value, such as 100 k$\Omega$ or 1 M$\Omega$. Based on Equation 10, the value of the resistor would be $R_3$=1k$\Omega$ ($R_3$ value may vary with the particular value of VCCS gain needed for each set of conditions, if increased detection sensitivity is required). Therefore, the output impedance of the current source is very high, and even for typical operational amplifier with CMRR of 60dB, the output impedance would "degrade" to 10 M$\Omega$ (acceptable value for applications in liquids or biomedicine). The majority of commercial operational amplifiers have CMRR (Common Mode Rejection Ratio) better than 80dB, so the impedance is increased even more. This circuit can supply many milliamperes (or as low as microamperes) depending on the input voltage and VCCS gain. Also, with a power supply of at $\pm$12V DC (nominal voltage values), the maximum resulting voltages caused by the output current, can be as large as 10 V, with good efficiency. If higher resulting voltages are needed, the power supply voltage can be increased. Based on the design guidelines of Equations 11 and 12 as well as on the gain requirement of Equation 10, the resistor values selected are those shown in Figure 5. The operation amplifier is a TL071 (but any other compatible operation amplifier can be used) JFET operational amplifier, with internal frequency compensation, high CMRR 100dB, high slew rate 13V/$\mu$s, low noise and distortion 0,003% (typical). Test measurements have shown that even for the case of a high value 1k$\Omega$ load, the gain is dropped only about 0.4% and a phase shift of -5,7° is observed. But these deviations are negligible and do not affect the accuracy of the measurements because when applying the system, a baseline measurement (bubble free condition) is always taken. Therefore, these changes are known and taken into account. The measured impedance (or inversely measured admittance) as "sensed" by the electrodes E1 and E2 (Fig. 5), is the

magnitude of $Z_m$ and can be simply calculated as the ratio of the differential voltage (voltage drop) between E1-E2 and output current:

$$Z_m = \frac{V_{E1} - V_{E2}}{I_{out}} = \frac{V_m}{I_{out}} \qquad (13)$$

If the phase requirements of Equation 8 are fulfilled, then $Z_m$ is transformed to resistance, i.e. $R_m$, similarly to the case of voltage source mode.

The voltages $V_{E1}$ and $V_{E2}$ can be measured either in single ended or differential mode. In single ended mode, the voltages measured by the data acquisition card are measured with respect to reference potential point. The most common setup is the 0V point, or in other words the ground reference. In differential mode, the voltage difference between two potential points is measured, such as the voltage drop across a resistor. The data acquisition card can measure voltages in both differential and single ended mode and is connected to a personal computer (PC) for real time display (monitoring) and digital signal processing of the measured voltages as well. The voltage difference $V_{E1}$-$V_{E2}$ is actually the measured key parameter ($I_{out}$ is constant), because it is directly affected by the variation of two-phase's impedance (caused by the presence of bubbles). Given that in differential mode the voltage drop is measured, differential set-up seems to be a more straight forward approach for the specific application. In addition, the differential mode provides better noise and interference rejection, but it requires the front-end electronic of the data acquisition card to be carefully designed with very high and well-matched input impedances as well as op-amps with high CMRR (more than 80dB). For the measurement setup, also, it is required to separate the current wires from the voltage measurement wires. Therefore, two extra cables for the voltage measurement are needed (4-wire measurement type). A typical application is shown in Fig. 6, where ring electrodes are flush mounted to the inner walls of a cylindrical pipe. The current excitation is accomplished via electrode E1, while electrode E2 is used for the voltage drop measurement.

The excitation and output voltages for the voltage source mode ($V_{E1}$ and $V_{E2}$), as well as the voltage drop for the current source mode ($V_{E1}$ - $V_{E2}$) are recorded by the data acquisition card of high sampling rate (192kS/s) and resolution (24bit), providing two important advantages: a) study of electrical signals with maximum frequency in the order of several tens of kHz due to the high sampling rate and b) measurement of extremely low voltages down to microvolts due to high spatial resolution. None of the conventional electrical techniques combine the aforementioned advantages. With the present technique, the measurement sensitivity is improved several orders of magnitude, providing detection capability of pretty low volumetric gas fraction values of ~0,001%. Thus, it is possible to detect extremely small variations due to the presence of a few micro-bubbles with unprecedented sensitivity. In addition, there is no need to calibrate the output with respect to known resistors since both $V_{E1}$ and $V_{E2}$ are measured and $R_t$ (for voltage source mode) as well as $R_3$ (for the current source mode) are known. Calibration procedure is often non-linear and thus induces measurement errors which may not be negligible for the case of applications that require capturing of extremely low voltage variations.

Next, the way that the acquired signals are digitally processed employing custom-made software, that complements the hardware of the innovative electrical impedance technique, is thoroughly described. When conducting measurements of low amplitudes, it is essential to reject unwanted interference such as power line 50 Hz related noise. Therefore, the recorded AC signals $V_{E1}$ and $V_{E2}$ (or $V_{E1}$ - $V_{E2}$) are digitally filtered, applying a very selective digital band pass filter centred at the excitation frequency with a nominal 3 kHz narrow bandwidth. Consequently, all kinds of electrical interference are effectively rejected, resulting in high quality electrical signals regardless of liquid physical properties, phase velocities and bubble sizes. If needed, the bandwidth can be set at any desired value, depending on the application, by changing the program code. Using a narrow filter bandwidth not only eliminates the 50 Hz related noise but also practically suppresses all extraneous noise outside the band pass frequency range. This reduces the total noise power and makes it possible to accurately measure very low voltage differentials, down to a few $\mu$V. The next critical issue of signal processing refers to the accurate extraction of acquired signals' envelope and for that reason the envelope of the filtered AC signals is digitally extracted, without any signal loss. This process is non-linear and therefore creates high frequency spurious components, which are removed by applying a low pass filter with a nominal cut-off frequency of 100 Hz. This cut-off frequency is adequate for the majority of physical characteristics of two-phase flows, but it can be set at any desired value depending on the application. It should be emphasized that conventional electrical techniques employing so far analogue electronics, either do not have filters for electrical interference rejection or these filters are not effective and selective enough, introducing in this way signal losses that affect both the accuracy and sensitivity of measurements. The particular feature of strong resistance to any form of electrical interference renders this impedance technique innovative and much more reliable than existing techniques.

The low pass filtered envelope of the signals $V_{E1}$ and $V_{E2}$, or the differential voltage $V_{E1}$ - $V_{E2}$, constitutes the real peak voltage amplitude of the signals without any loss of information or distortion. Then, the impedance's magnitude of two-phase medium, $Z_m$, can be calculated employing Equations (2) and (13). Comparing $Z_m$ values prior and after gas phase entrance in the liquid phase enables the quantitative detection of bubbles inside the two-phase medium, while the resulted time series can be easily converted to volumetric gas fraction time series, for the case of a pure resistive medium -e.g. two-phase flow inside a pipe for a predefined excitation frequency-, employing standard conversion models such as Maxwell's and Begovich' ones. In any case, impedance time series carry crucial information in both frequency and time domain. Therefore, several statistical and spectral features can be associated with bubble size characteristics. As a result, various methods of signal processing and statistical analysis can be applied to the resulted time series, e.g.:

a) $1^{st}$, $2^{nd}$ and higher order moments: Mean value, standard deviation, coefficient of variation, skewness and kurtosis.
b) Fast Fourier Transform (FFT) spectral analysis algorithm.
c) Auto-correlation.
d) Cross-correlation.
e) Spectrogram analysis.
f) Continuous and discrete wavelets transform.
g) Hilbert Huang transform.

Depending on the application, e.g. in-vivo or in-vitro, different quantities determined by the above statistical and spectral tools such as skewness, standard deviation, power spectral density, dominant spectral frequency, or a combination of them, can provide for the first time valuable information on bubble size distribution, as well as to be correlated with bubble size. Moreover, a number of tools contribute to further study two-phase flow dynamics, e.g. the cross-correlation of the signals between two electrode pairs provides bubble velocity. It should be pointed out that the previously mentioned signal analysis techniques are not exclusive. Any digital signal processing technique or method can be applied on the impedance time series in order to study bubble size features for specific two-phase flow applications.
The most important advantage of the electrical impedance technique software concerns digital processing of the acquired signals. This processing can take place either in real time or at a later time (post-processing). Moreover, new processing features can be easily added by simply changing the program code. Computer technology advance in terms of storage capacity, memory and CPU processing power allows for the storage of numerous voluminous data files. The volume of the acquired files is determined by the recording duration which is dictated by the stochastic features of the studied two-phase flow. On the contrary, the conventional electrical techniques provide mostly predefined signal processing features which are accomplished by analogue electronics, while the storing capacity is limited by the built-in memory, which in many cases cannot be upgraded, and post-processing abilities are limited by the installed electronics components and processors.

## Claims

1. Method of electrical impedance spectroscopy for non-invasive bubbles detection as well as estimation of their size corresponding gas volumetric fraction, in a two phase medium present in a human body, wherein said method comprises the steps of:

a) attaching at least two non-invasive sensors consisting of Electrocardiography (ECG) or Electroencephalography (EEG) electrodes pads, to said human body, in a manner to obtain electrical impedance measurements;
b) generating an electrical signal using a voltage source excitation mode or a current source excitation mode and applying the generated signal on the at least two non-invasive electrodes;
c) measuring the applied signal in a single ended or a differential mode with respect to a reference point;
d) recording the measured signal by means of a data acquisition means, consisting of a data acquisition card having a sampling rate and a resolution to detect variations generated by the presence of micro-bubbles in said two-phase medium;
e) real time monitoring, storing and digital processing of acquired signals, by means of a personal computer (PC);
f) calculating an impedance of the two phase mixing using the measured signal; and
g) detecting quantitatively and estimating size of said micro-bubbles inside the two-phase medium using the calculated impedance,
**characterized in that** the excitation and output voltages for the voltage source mode ($V_{E1}$ and $V_{E2}$), as well as the voltage drop for the current source mode ($V_{E1}$ - $V_{E2}$) are recorded by the said data acquisition card of sampling rate 192 kS/s and resolution 24 bit, wherein the measurement sensitivity is increased substantially, whereby low volumetric gas fraction values of down to 0,001% is detected, whereby extremely small variations generated by the presence of micro-bubbles is detected with increased sensitivity.

2. Method according to claim 1, **characterized in that** both said $V_{E1}$ and $V_{E2}$ are measured whereas $R_t$ for voltage source mode as well as $R_3$ for the current source mode are known.

3. Method according to claim 1, **characterised in that** said human body consists of astronauts, scuba divers, metro workers and patients treated in decompression chambers.

4. Method according to claim 1, **characterized in that** said non-invasive sensors are applied above a human skin for bubbles detection inside blood flow in the human body.

## Patentansprüche

1. Verfahren zur elektrischen Impedanzspektroskopie zur nicht-invasiven Bläschenfeststellung sowie Schätzung ihrer größenentsprechenden Gasvolumenfraktion in einem Zwei-Phasenmedium, das in einem menschlichen Körper vorhanden ist, wobei das Verfahren die Schritte umfasst:

a) Befestigen von wenigstens zwei nicht-invasiven Sensoren, die aus Elektrokardiographie- (EKG) oder Elektroenzephalographie- (EEG)-Elektroden-Pads gebildet werden, an dem menschlichen Körper derart, dass elektrische Impedanzmessungen erhalten werden;
b) Erzeugen eines elektrischen Signals unter Verwenden eines Spannungsquellen-Erregungsmodus oder eines Stromquellen-Erregungsmodus und Anwenden des erzeugten Signals auf die wenigstens zwei nicht-invasiven Elektroden;
c) Messen des angewendeten Signals nach einem unsymmetrischen oder einem symmetrischen Modus in Bezug auf einen Bezugspunkt;
d) Aufzeichnen des gemessenen Signals mittels eines Datenerfassungsmittels, das aus einer Datenerfassungskarte mit einer Abtastfrequenz und einer Auflösung gebildet wird, um Abweichungen zu erfassen, die vom Vorhandensein von Mikrobläschen in dem Zweiphasen-Medium erzeugt werden;
e) Echtzeitüberwachung, Speichern und digitales Verarbeiten von erfassten Signalen mittels eines Personalcomputers (PC);
f) Berechnen einer Impedanz der zwei Phasen-Mischung durch Verwenden des gemessenen Signals; und
g) quantitatives Erfassen und Schätzen der Größe der Mikro-Bläschen innerhalb des zwei Phasen-Modus unter Verwenden der berechneten Impedanz,

**dadurch gekennzeichnet, dass** die Erregungs- und Wiedergabespannungen für den Spannungsquellen-Modus ($V_{E1}$ und $V_{E2}$) sowie der Spannungsabfall für den Stromquellen-Modus ($V_{E1}$ - $V_{E2}$) von der Datenerfassungskarte mit einer Abtastfrequenz 192 kS/s und Auflösung von 24 Bit aufgezeichnet werden, wobei die Messungssensibilität im Wesentlichen erhöht ist, wobei niedrige Volumengas-Fraktionswerte bis hinab zu 0,001 % erfasst werden, wodurch extrem geringe Abweichungen, die durch das Vorhandensein von Mikro-Bläschen erzeugt werden, mit erhöhter Sensibilität erfasst werden.

2. Verfahren nach Anspruch 1, **dadurch gekennzeichnet, dass** sowohl $V_{E1}$ als auch $V_{E2}$ gemessen werden, wohingegen Rt für den Spannungsquellen-Modus sowie $R_3$ für den Stromquellen-Modus bekannt sind.

3. Verfahren nach Anspruch 1, **dadurch gekennzeichnet, dass** der menschliche Körper aus Astronauten, Gerätetauchern, U-Bahnarbeitern und Patienten gebildet wird, die in Dekompressionskammern behandelt werden.

4. Verfahren nach Anspruch 1, **dadurch gekennzeichnet, dass** die nicht-invasiven Sensoren oberhalb einer menschlichen Haut zur Bläschenerfassung innerhalb des Blutstroms im menschlichen Körper angewendet werden.

## Revendications

1. Procédé de spectroscopie d'impédance électrique pour la détection non invasive de bulles ainsi que l'estimation de leur fraction volumétrique de gaz correspondant en taille, dans un milieu biphasique présent dans le corps humain, comprenant les étapes suivantes :

a) fixation d'au moins deux capteurs non invasifs, constitués d'électrodes d'électrocardiographie (ECG) ou d'électroencéphalographie (EEG), audit corps humain, de façon à obtenir des mesures d'impédance électrique ;
b) génération d'un signal électrique par mode d'excitation par source de tension ou par mode d'excitation de courant, et application du signal généré au moins aux deux électrodes non invasives ;
c) mesure du signal appliqué en mode asymétrique ou différentiel par rapport à un point de référence ;

d) enregistrement du signal mesuré au moyen d'un moyen d'acquisition de données, constitué d'une carte d'acquisition de données dont la fréquence d'échantillonnage et la résolution permettent de détecter les variations générées par la présence de microbulles dans le milieu biphasique précité ;

e) surveillance, stockage et traitement numérique en temps réel des signaux acquis, au moyen d'un ordinateur personnel (PC) ;

f) calcul de l'impédance du mélange biphasé à partir du signal mesuré ; et

g) détection quantitative et estimation de la taille des microbulles présentes dans le milieu biphasé à partir de l'impédance calculée,

**caractérisé en ce que** les tensions d'excitation et de sortie pour le mode source de tension ($V_{E1}$ et $V_{E2}$), ainsi que la chute de tension pour le mode source de courant ($V_{E1}$ - $V_{E2}$) sont enregistrées par ladite carte d'acquisition de données à une fréquence d'échantillonnage de 192 kS/s et une résolution de 24 bits, la sensibilité de mesure étant considérablement accrue, permettant de détecter de faibles valeurs de fraction volumique de gaz, jusqu'à 0,001 %, et de détecter de très faibles variations générées par la présence de microbulles avec une sensibilité accrue.

2. Procédé selon la revendication 1, **caractérisé en ce que** les deux $V_{E1}$ et $V_{E2}$ sont mesurés, tandis que Rt pour le mode source de tension et $R_3$ pour le mode source de courant sont connus.

3. Procédé selon la revendication 1, **caractérisé en ce que** ledit corps humain est constitué d'astronautes, de plongeurs, d'employés du métro et de patients traités en chambres de décompression.

4. Procédé selon la revendication 1, **caractérisé en ce que** lesdits capteurs non invasifs sont appliqués au-dessus de la peau humaine pour détecter la présence de bulles à l'intérieur du flux sanguin dans le corps humain.

FIG. 1

FIG. 2

FIG. 3

FIG. 4

FIG. 5

FIG. 6

**EP 3 005 942 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6813944 B2
- US 7661293 B
- US 7661294 B
- WO 9965390 A